# FASCICULE DE BREVET EUROPEEN

(11) **EP 0 490 793 B1**
(45) Date de publication et mention de la délivrance du brevet: **22.05.1996**
(21) Numéro de dépôt: 91420439.1
(22) Date de dépôt: 10.12.1991
(51) Int. Cl.: A61F 13/00

(54) **Bande de contention avec moyen d'étalonnage**
Kompressionsbandage mit Normungsmittel
Compression bandage with standardization means

(30) Priorité: 11.12.1990 FR 9015877
(43) Date de publication de la demande: 17.06.1992
(73) Titulaire: TOPIC DIFFUSION SA, CH-2072 SAINT-BLAISE (CH)
(72) Inventeur: Delannoy Robert, 75016 - Paris (FR)
(74) Mandataire: Derambure, Christian

(56) Documents cités:
- DE-A- 2 329 371
- DE-A- 3 640 979
- FR-A- 2 544 982
- GB-A- 2 180 161

## Description

Plus particulièrement, l'invention concerne les bandes de contention ayant une capacité d'étirage dans le sens longitudinal et éventuellement transversal, destinées à être enroulées, généralement d'une manière hélicoïdale autour d'un membre.

En fonction des cas pathologiques à traiter, il est très important de pouvoir connaître la force de pression exercée par la bande sur le membre, au fur et à mesure de son enroulement, compte-tenu de l'élasticité de ladite bande.

Pour résoudre ce problème, on a proposé des bandes de contention comportant des moyens constituant un étalonnage pour l'utilisateur. Par exemple, cet état de la technique peut être illustré par l'enseignement des brevets américain 3 613 679 et français 2 544 982.

Le brevet américain 3 613 679 enseigne une bande de contention présentant, très sensiblement dans sa partie centrale, en étant régulièrement répartis sur la totalité de sa longueur, des repères de formes géométriques variables. La mesure du degré d'allongement de la bande et par conséquent, la pression en résultant exercée sur le membre, s'effectue visuellement par une déformation des repères géométriques.

Le brevet français 2 544 982 enseigne une solution équivalente à celle divulguée par le brevet américain, c'est-à-dire où le degré d'allongement de la bande est mesuré par une déformation de repères que présente ladite bande. Plus particulièrement, dans le brevet français 2 544 982, le problème est résolu en imprimant sur la bande, très sensiblement dans sa partie médiane, deux rangées parallèles de points disposés à intervalles réguliers. Les deux rangées son séparées d'une distance supérieure à l'intervalle séparant deux points d'une même rangée. Lorsque l'on étire la bande, l'intervalle séparant deux points, varie, en pouvant atteindre la longueur de la distance séparant les deux rangées, ce qui correspond à un certain pourcentage d'allongement.

On connaît également le brevet allemand 2329371 qui enseigne une bande de contention avec dispositif indicateur de l'état d'allongement de la bande et indirectement de la pression exercée. Il est par ailleurs prévu d'équiper la bande, de dispositifs indicateurs qui peuvent être différents pour correspondre chacun à un certain degré d'allongement. Cependant, de tels dispositifs indicateurs sont disposés selon des intervals constants, de sorte qu'il est seulement possible d'obtenir une indication ponctuelle. Enfin, il convient de souligner qu'il est difficilement envisageable de réaliser d'une manière industrielle, une bande de contention équipée à différents endroits, d'indicateurs visuels de formes de réalisations différentes.

Il apparait donc que si les solutions enseignées par l'un ou l'autre de ces documents permettent de visualiser le degré d'allongement de la bande, la pression exercée, en fonction de ce degré d'allongement, est toujours constante. En effet, les repères visuels, quelles que soient leurs formes géométriques, sont séparés par des intervalles constants sur toute la longueur de la bande.

Or, il est médicalement prouvé, notamment en phlébologie, que la pression exercée par la bande, depuis le pied en remontant vers la cuisse, doit être décroissante. A défaut, certains traumatismes peuvent apparaître.

L'invention s'est fixée pour but de remédier à ces inconvénients de manière simple et efficace.

Le problème que se propose de résoudre l'invention est de pouvoir contrôler et diminuer la pression exercée par la bande sur le membre, au fur et à mesure de son enroulement.

Un tel problème est résolu en ce que la bande présente des repères visuels disposés selon un écartement variable et progressivement croissant pour correspondre à une pression exercée dégressive, au fur et à mesure de l'enroulement de la bande.

Pour résoudre le problème posé de disposer les repères visuels selon un écart variable et progressif, différentes solutions peuvent être envisagées.

Dans une forme de réalisation, les repères sont disposés selon plusieurs séries, pour chaque série, lesdits repères étant écartés d'un pas différent, le pas de chacune de ces séries étant progressivement croissant.

Dans une autre forme de réalisation, les repères sont disposés selon un écartement variable et progressivement croissant, d'une manière continue.

Avantageusement, compte tenu du problème posé, les repères sont disposés sur la bande par jet d'encre, sans pour cela exclure toute autre technique permettant d'obtenir un résultat similaire.

Les repères sont disposés en alignement selon le sens d'allongement de la bande et d'une manière parallèle aux lisières.

Un autre problème que se propose de résoudre l'invention, est de rendre invisibles les repères au fur et à mesure de l'enroulement de la bande. Dans les techniques connues, telles que rappelées précédemment, après positionnement de la bande de contention sur le membre, les repères d'étalonnage sont visibles, ce qui n'est pas très esthétique.

Un tel problème est résolu en ce que les repères sont disposés en lisière ou à proximité de l'une des lisières.

Suivant une autre caractéristique, les repères sont de forme géométrique quelconque. A noter que ces formes géométriques peuvent indiquer le sens de pose de la bande.

L'invention est exposée, ci-après, plus en détail à l'aide des dessins annexés, dans lesquels :
- la figure 1 est une vue en plan de la bande, selon une première forme de réalisation ;
- la figure 2 est uen vue semblable à la figure 1, montrant une autre forme de réalisation de la bande ;
- la figure 3 est une vue en perspective montrant l'enroulement de la bande de contention selon l'invention au niveau du membre inférieur;
- les figures 4,5,6 et 7 sont des vues à caractère purement schématique, montrant le principe d'étalonnage, selon l'invention, et la diminution de la pression exercée, au fur et à mesure de l'enroulement de la bande.

La bande de contention, utiliseé dans le procédé selon l'invention, est de tout type connu et approprié, résultant d'un entrelacement de fils de chaîne et de trame, tissés ou tricotés, de nature et de contexture déterminées pour conférer à la bande une certaine élasticité, notamment dans le sens longitudinal. Cette bande est désignée, dans son ensemble, par (B).

Selon une caractéristique à la base de l'invention, la bande (B) présente des repères visuels (1) disposés selon un écartement variable et progressivement croissant.

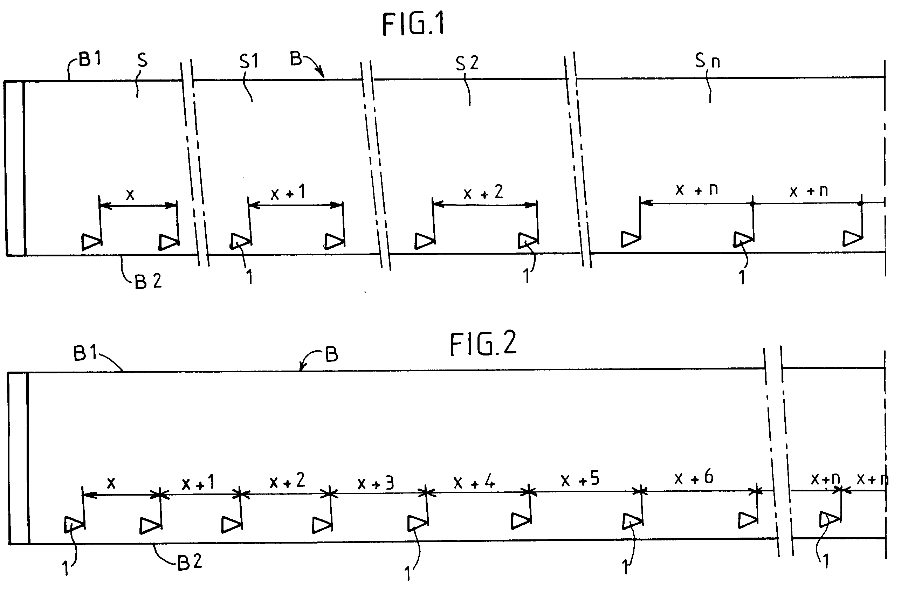

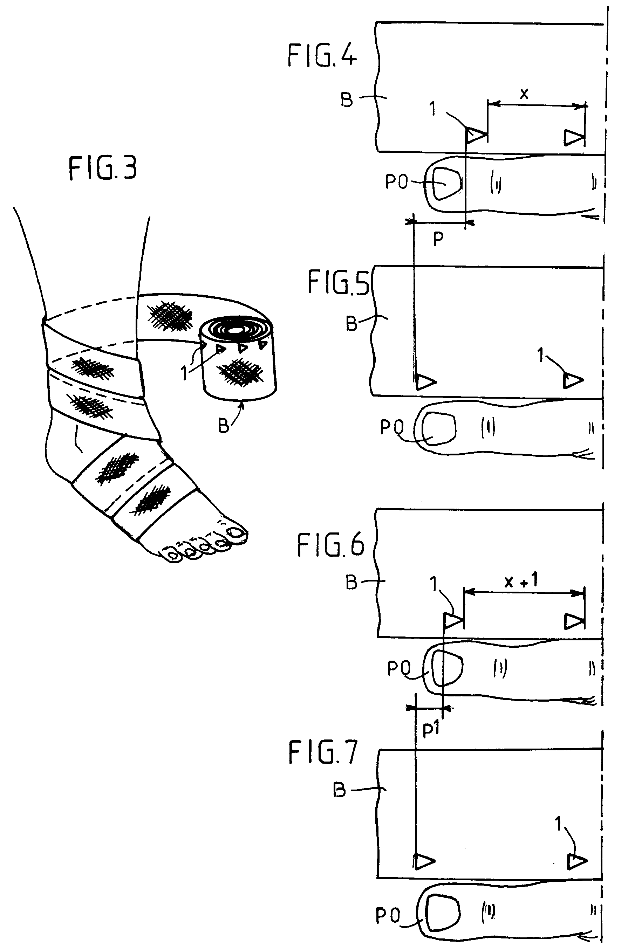

Dans la forme de réalisation illustrée à la figure 1, les repères (1) sont disposés selon plusieurs séries (S), (S1), (S2), ... (Sn) comprenant chacune un certain nombre de repères. Pour chaque série, les repères (1) sont écartés d'un pas différent, les pas de chacune de ces séries, étant croissants. Par exemple, si les repères de la première série (S) sont espacés d'un pas (x), ceux de la seconde série (S1) seront espacés d'un pas (x + 1), ceux de la troisième série (S2), d'un pas (x + 2) et ainsi de suite, jusqu'à l'extrémité de la bande, où les repères de la derniière série (Sn) seront espacés d'un pas (x + n).

Dans la forme de réalisation illustrée à la figure 2, les repères (1) sont disposés entre chaque extrémité de la bande, selon un écartement variable (x), (x + 1), (x + 2), ... (x + n) et progressivement croissant, d'une manière continue.

Les différents repères (1) sont disposés dans les conditions indiquées précédemment, c'est-à-dire selon un écartement variable et progressivement croissant, en alignement selon le sens d'allongement de la bande et d'une manière parallèle aux lisiières (B1) (B2).

Compte tenu de ces dispositions, il est possible de contrôler la pression exercée par la bande (B) sur le membre, au fur et à mesure de son enroulement. D'une manière particulièrement avantageuse, il apparait que la pression exercée est dégressive, au fur et à mesure de l'enroulement de la bande, résultant de l'écartement variable et progressivement croissant, des repères visuels d'étalonnage (1).

Par exemple, si au moment de l'enroulement de la bande sur le membre, cette dernière est étirée de sorte qu'après extension, les repères correspondent très sensiblement à la longueur du pouce (PO), on conçoit, qu'au fur et à mesure de l'enroulement, la bande sera étirée d'une valeur moindre, pour que la distance entre deux repères corresponde toujours à la longueur du pouce (PO) qui est invariable, en constituant la base de l'étalonnage. Bien évidemment, le repère d'étalonnage constitué par le pouce, est donné seulement à titre indicatif, nullement limitatif, étant bien entendu, qu'il est possible d'employer tout moyen de mesure.

On a illustré aux figures 4, 5, 6 et 7, par des vues à caractère purement schématique, le principe de contrôle de la tension de la bande selon l'invention.

La figure 4 montre une partie de la bande au niveau de son extrémité d'enroulement, où, par exemple, les repères (1) sont séparés d'un intervalle (x). En regard de ces repères, on a schématisé le pouce (PO) de l'utilisateur. A ce stade, la bande est représentée à l'état repos, c'est-à-dire avant allongement.

La figure 5 est une vue correspondant à la figure 4, montrant l'allongement de la bande, pour disposer les repères (1) selon la longueur du pouce (PO).

En comparant ces deux figures, on voit qu'il a été nécessaire d'allonger la bande, selon une valeur (P) correspondant à une certaine pression.

La figure 6 montre une autre partie de la bande où les repères (1) sont séparés d'un intervalle (x + 1). De la même façon que précédemment, on a représenté, en regard de ces repères, le pouce (PO) de l'utilisateur. La bande est représentée avant allongement.

A la figure 7, la bande est étirée pour disposer les repères (1) selon la longueur du pouce (PO). Si l'on compare comme précédemment, les figures 6 et 7, on voit qu'il a été nécessaire d'allonger, dans ce cas, la bande selon une pression (P1), inférieure à la pression (P).

Un autre problème que se propose de résoudre l'invention est de rendre invisibles les différents repères (1), au fur et à mesure de l'enroulement de la bande.

Dans ce but, les repères (1) sont disposés en lisière ou à proximité de l'une des lisières (B1) (B2) de la bande. Dans ces conditions, compte tenu de l'enroulement hélicoïdal de la bande et du chevauchement partiel des différentes spires, il apparait que les repères sont automatiquement cachés par les parties en recouvrement de la bande.

Avantageusement, les différents repères (1) sont obtenus par la technique connue du jet d'encre, par tout moyen approprié, permettant de positionner ces repères avec un écartement variable et progressivement croissant. Là encore, cette technique d'impression, par jet d'encre, constitue seulement un exemple préféré de réalisation, étant donné que les repères peuvent être inscrits sur la bande par toute technique permettant d'obtenir des résultats similaires.

Aux figures des dessins, les repères illustrés sont de forme géométrique triangulaire, sans pour cela exclure, d'autres formes géométriques. Ces formes géométriques peuvent indiquer avantageusement le sens de pose de la bande.

Les avantages ressortent bien de la description, en particulier on souligne et on rappelle :
- la possibilité de contrôler la pression exercée par la bande sur le membre, tout en permettant de diminuer cette pression, au fur et à mesure de son enroulement;
- la simplicité de réalisation ;
- l'invisibilité des repères d'étalonnage, après enroulement de la bande.

## Revendications

1. Procédé consistant à inscrire et à disposer sur une bande de contention des repères visuels (1) positionnés selon un écartement variable et progressivement croissant (x), (x+1), (x+2)... (x+n), la bande présentant ces repères visuels (1) permettant le contrôle et la diminution de la pression exercée sur un membre sur lequel elle est enroulée, au fur et à mesure de son enroulement.

2. Procédé selon la revendication 1, caractérisé en ce que les repères (1) sont disposés selon plusieurs séries (S), (S1), (S2)... (Sn), pour chaque série, ledits repères (1) sont écartés d'un pas différent (x), (x+1), (x+2)... (x+n), le pas de chacune de ces séries étant progressivement croissant.

3. Procédé selon la revendication 1 caractérisé en ce que les repères (1) sont disposés selon un écartement variable et progressivement croissant (x), (x+1), (x+2)... (x+n), de manière continue.

4. Procédé selon l'une quelconque des revendications 1 à 3, caractérisé en ce que les repères (1) sont disposés sur la bande par jet d'encre notamment.

5. Procédé selon l'une quelconque des revendications 1 à 4, caractérisé en ce que les repères (1) sont disposés en alignement selon le sens d'alignement d'allongement de la bande et de manière parallèle aux lisières (B1), (B2) de la bande.

6. Procédé selon la revendication 5, caractérisé en ce que les repères (1) sont disposés en lisière ou à proximité de l'une des lisières (B1) de la bande.

7. Procédé selon l'une quelconque des revendications 1 à 6, caractérisé en ce que les repères (1) sont de forme géométrique quelconque.

## Claims

1. A method consisting of marking and setting out, on a retention strip, visual markers (1) positioned according to a varying, progressively increasing spacing (X), (X+1), (X+2) ... (X+n), the strip displaying these visual markers (1) making it possible to check and to decrease the pressure exerted on a limb around which it is wrapped, as it is wrapped.

2. A method according to claim 1, characterised in that the markers (1) are set out according to various series (S), (S1), (S2) ... (Sn), said markers (1) being separated by a different pitch (X), (X+1), (X+2) ... (X+n) for each series, with the pitch of each of these series progressively increasing.

3. A method according to claim 1, characterised in that the markers (1) are set out according to a varying, progressively increasing spacing (X), (X+1), (X+2) ... (X+n), in a continuous manner.

4. A method according to any of claims 1 to 3, characterised in that the markers (1) are set out on the strip, particularly by an ink jet.

5. A method according to any of claims 1 to 4, characterised in that the markers (1) are set out in alignment according to the direction of lengthwise alignment of the strip and parallel to the edges (B1), (B2) of the strip.

6. A method according to claim 5, characterised in that the markers (1) are set out on or near one of the edges (B1) of the strip.

7. A method according to any of claims 1 to 6, characterised in that the markers (1) have any geometric shape whatsoever.

## Patentansprüche

1. Verfahren, darin bestehend, daß auf einem Druckverband Sichtmarkierungen (1) eingezeichnet und angeordnet werden, welche in veränderlichem und progressiv zunehmendem Abstand (x), (x+1), (x+2) ... (x+n) positioniert sind, wobei mit dem diese Sichtmarkierungen (1) aufweisenden Verband der Druck, der auf ein Glied ausgeübt wird, auf welches er gewickelt ist, bei fortschreitender Wicklung geprüft und vermindert werden kann.

2. Verfahren nach Anspruch 1, dadurch gekennzeichet, daß die Markierungen (1) in mehreren Serien (S), (S1), (S2) ... (Sn) angeordnet sind, wobei die Markierungen (1) jeder Serie durch einen unterschiedlichen Schritt (X), (X+1), (X+2) ... (x+n) voneinander beabstandet sind, wobei der Schritt jeder dieser Serien progressiv zunimmt.

3. Verfahren nach Anspruch 1, dadurch gekennzeichnet, daß die Markierungen (1) durchlaufend in veränderlichem und progressiv zunehmendem Abstand (x), (x+1), (x+2) ... (x+n) angeordnet sind.

4. Verfahren nach einem der Ansprüche 1 bis 3, dadurch gekennzeichnet, daß die Markierungen (1) insbesondere durch Tintenstrahl auf dem Verband angeordnet sind.

5. Verfahren nach einem der Ansprüche 1 bis 4, dadurch gekennzeichnet, daß die Markierungen (1) auf die Dehnungsrichtung des Verbands ausgerichtet und parallel zu den Rändern (B1), (B2) des Verbands angeordnet sind.

6. Verfahren nach Anspruch 5, dadurch gekennzeichnet, daß die Markierungen (1) am Rand oder in der Nähe einer der Ränder (B1) des Verbands angeordnet sind.

7. Verfahren nach einem der Ansprüche 1 bis 6, dadurch gekennzeichnet, daß die Markierungen (1) eine beliebige geometrische Form aufweisen.
